# EUROPÄISCHE PATENTANMELDUNG

(11) **EP 0 928 621 A2**
(43) Veröffentlichungstag der Anmeldung: **14.07.1999**
(21) Anmeldenummer: 98123623.5
(22) Anmeldetag: 10.12.1998
(51) Int. Cl.: A61M 39/26

(54) **Schlauchkupplung für ein medizinisches Überleitungssystem**

(30) Priorität: 07.01.1998 DE 29800107 U
(71) Anmelder: B. BRAUN MELSUNGEN AG, 34212 Melsungen (DE)
(72) Erfinder: Fuchs, Jürgen, 34308 Bad Emstal (DE)
(74) Vertreter: Selting, Günther, Dipl.-Ing.

(57) **Zusammenfassung**

Die Schlauchkupplung enthält ein Kupplungselement (14), das einen Durchflußkanal (20) aufweist, und ein Steckerteil (15) mit einem Steckansatz (42), welcher in den Durchflußkanal des Kupplungselements (14) einsteckbar ist. Der Steckansatz (42) öffnet im eingesteckten Zustand das Ventil (21). Zum Festhalten des Steckerteils (15) am Kupplungselement (14) dienen Rastklinken (46), die jeweils mit einer Rastnase (47) hinter einem Halteteil (26) einrasten. Dadurch wird das Abziehen des Steckerteils (15) blockiert. Das Steckerteil (15) kann eine zweite Position einnehmen, bei der der Steckansatz (42) das Ventil (21) noch nicht aufstößt, jedoch bereits abdichtend in dem Durchflußkanal sitzt. Dadurch kann auf zusätzliche Schlauchklemmen verzichtet werden.

## Beschreibung

Die Erfindung betrifft eine Schlauchkupplung für ein medizinisches Überleitungssystem, beispielsweise für die Entnahme medizinischer Lösungen aus einem Beutel oder einer Flasche.

Zur Entnahme von Flüssigkeiten aus einem Beutel oder einem anderen Behältnis ist es üblich, an dem Behältnis einen Auslaßstutzen vorzusehen, der eine Membran in Form eines Gummistopfens enthält. Diese Membran wird mit dem Einstechdorn eines Überleitungsgerätes durchstochen. Durch einen im Einstechdorn vorgesehenen Kanal fließt die Flüssigkeit aus dem Behälter in den Einstechdorn und einen damit verbundenen Schlauch. Einstechdorne haben eine Reihe von Nachteilen. So erfordert das Durchstechen der Membran mit dem Einstechdorn einen erheblichen Kraftaufwand. Wegen der Spitze des Einstechdornes bestehen Verletzungsgefahren. Außerdem können durch den Einstechdorn Partikel aus der Membran freigesetzt werden und in die Flüssigkeit gelangen. Vorteilhaft ist, daß die Membran nach dem Herausziehen des Einstechdornes wieder dicht verschließt.

Darüber hinaus sind Schlauchkupplungen für medizinische Überleitungssysteme bekannt, die mit Luer-Lock-Verschlüssen ausgestattet sind und eine Dekonnektierung ermöglichen. Solche Schlauchkupplungen lassen allerdings keine Rotationsbewegungen am Überleitungssystem zu, weil dann die Gefahr der unbeabsichtigten Dekonnektierung besteht.

Der Erfindung liegt die Aufgabe zugrunde, eine Schlauchkupplung für ein medizinisches Überleitungssystem zu schaffen, die ohne einen Einstechdorn auskommt, beim Dekonnektieren wieder dicht verschließt und durch Rotationsbewegungen am Überleitungssystem nicht geöffnet werden kann.

Die Lösung dieser Aufgabe erfolgt erfindungsgemäß mit einer Schlauchkupplung, die die Merkmale des Patentanspruchs 1 aufweist.

Die erfindungsgemäße Schlauchkupplung besteht aus einem einen Durchflußkanal enthaltenden Kupplungselement, an das ein Steckerteil ankuppelbar ist. Das Kupplungselement enthält ein Ventil, welches von einem Steckansatz des eingesteckten Steckerteils aufgedrückt wird. An dem Kupplungselement ist ein Halteteil vorgesehen, welches das Einrasten einer Rastvorrichtung des Steckerteils dann ermöglicht, wenn der Steckansatz das Ventil geöffnet hält. Das Steckerteil rastet also in der Öffnungsstellung des Ventils an dem Kupplungselement ein, so daß es nicht ohne besondere Maßnahmen wieder gelöst werden kann. Infolge des Einrastens wird das Steckerteil an dem Kupplungselement festgehalten, auch wenn das Überleitungssystem gedreht wird, beispielsweise durch Verwinden der entsprechenden Schlauchleitungen. Durch solche Drehungen kann der Kupplungseingriff nicht gelöst werden. Das Lösen des Kupplungseingriffs erfordert zwingend eine manuelle Betätigung einer Lösevorrichtung. Wichtig ist, daß das Steckerteil an dem Kupplungselement schnappend einrastet, daß also das Ankuppeln nicht lediglich durch Klemmung erfolgt, sondern durch Überschnappen eines Rastelements über ein Halteteil. Damit ist die Schlauchkupplung gegen unbeabsichtigtes Lösen gesichert.

Die erfindungsgemäße Schlauchkupplung läßt sich kostengünstig aus Kunststoff-Spritzgußteilen herstellen. Es sollten Kunststoffmaterialien auf polyolephinischer Basis verwendet werden.

Vorzugsweise ist das erstes Halteteil ein Ringkragen, der mindestens eine von der Rastvorrichtung überwindbare Einführschräge aufweist und das Zurückziehen der Rastvorrichtung verhindert. Infolge des Ringkragens kann das Steckerteil relativ zu dem Kupplungselement beliebig gedreht werden, ohne daß die Kupplungsverbindung aufgehoben oder undicht wird.

Gemäß einer bevorzugten Weiterbildung der Erfindung ist an dem Kupplungselement ein zweites Halteteil vorgesehen, welches das Einrasten der an dem Steckerteil vorgesehenen Rastvorrichtung dann ermöglicht, wenn der Steckeransatz abdichtend in dem Durchflußkanal sitzt, das Ventil jedoch noch nicht aufstößt. Hierdurch ist es möglich, das Steckerteil zunächst vorbereitend mit dem Kupplungselement zu verbinden, ohne daß ein Flüssigkeitsdurchfluß bereits stattfindet. Es besteht daher keine Notwendigkeit in dem Schlauch des Überleitungssystems eine Schiebeklemme oder ein anderes Absperrorgan vorzusehen, um zu verhindern, daß unmittelbar nach Herstellung der Verbindung zwischen Überleitungssystem und Infusionsbehälter der Flüssigkeitsstrom beginnt. Das Steckerteil hat an dem Kupplungselement zwei Raststellungen, wobei in beiden Raststellungen der Durchgang zwischen Durchflußkanal und Steckerteil hermetisch nach außen abgedichtet ist. Beide Stellungen unterscheiden sich jedoch dadurch, daß in der einen Stellung das Ventil geöffnet und in der anderen Stellung das Ventil geschlossen ist.

Grundsätzlich besteht die Möglichkeit, die Rastung nach Art einer Kugelraste vorzunehmen, wobei eine gewisse Grenzkraft überwunden werden muß, um die Rastverbindung zu lösen, bzw. von einer Raststellung in die andere zu gelangen. Vorzugsweise ist jedoch die Rastvorrichtung derart ausgebildet, daß sie mit einer Rastklinke an einer Sägezahnstruktur angreift, wobei in Aufschieberichtung der Rastklinke die Sägezahnstruktur automatisch überwunden wird, während in Abzugsrichtung die Sägezahnstruktur das Zurückziehen der Rastklinke verhindert. Durch Betätigung eines Auslösehebels kann die Rastvorrichtung zum Zurückziehen freigegeben werden. Dies hat den Vorteil einer zusätzlichen Sicherheit gegen unbeabsichtigtes Lösen durch Auseinanderziehen. Diese Sicherheit wird noch erhöht, wenn die Rastvorrichtungen zwei Rastklinken mit jeweils einem eigenen Auslösehebel aufweist. In diesem Fall müssen zum Lösen der Rastverbindung beide Auslösehebel gleichzeitig betätigt sein.

Um zu verhindern, daß beim Ankuppeln des Steckerteils an das Kupplungselement versehentlich die falsche Rastposition eingestellt wird, können die Halteteile jeweils mindestens eine von der Rastvorrichtung überwindbare Einführschräge aufweisen, wobei die Einführschräge des einen Halteteils zu der Einführschräge des anderen Halteteils umfangsmäßig versetzt ist. Dies hat zur Folge, daß durch eine axiale Einschubbewegung nur eine Einrastung in der Bereitschaftsposition möglich ist. Um die Betriebsposition, d.h. die Öffnungsposition des Ventils, einzustellen, muß das Steckerteil gedreht und anschließend weiter aufgeschoben werden.

Im folgenden wird unter Bezugnahme auf die Zeichnungen ein bevorzugtes Ausführungsbeispiel der Erfindung näher erläutert.

Es zeigen:
- Fig. 1: einen ein Spüllösung enthaltenden Beutel, an den ein Überleitungssystem angekuppelt ist,
- Fig. 2: die beiden Komponenten der Schlauchkupplung im getrennten Zustand, jeweils mit einer Schutzkappe versehen,
- Fig. 3: die Schlauchkupplung im Kupplungszustand in der Bereitschaftsstellung,
- Fig. 4: eine stirnseitige Ansicht des Endes des Kupplungselements aus Richtung des Pfeiles IV von Fig. 3, und
- Fig. 5: die Schlauchkupplung in der Betriebsposition, mit offenem Ventil.

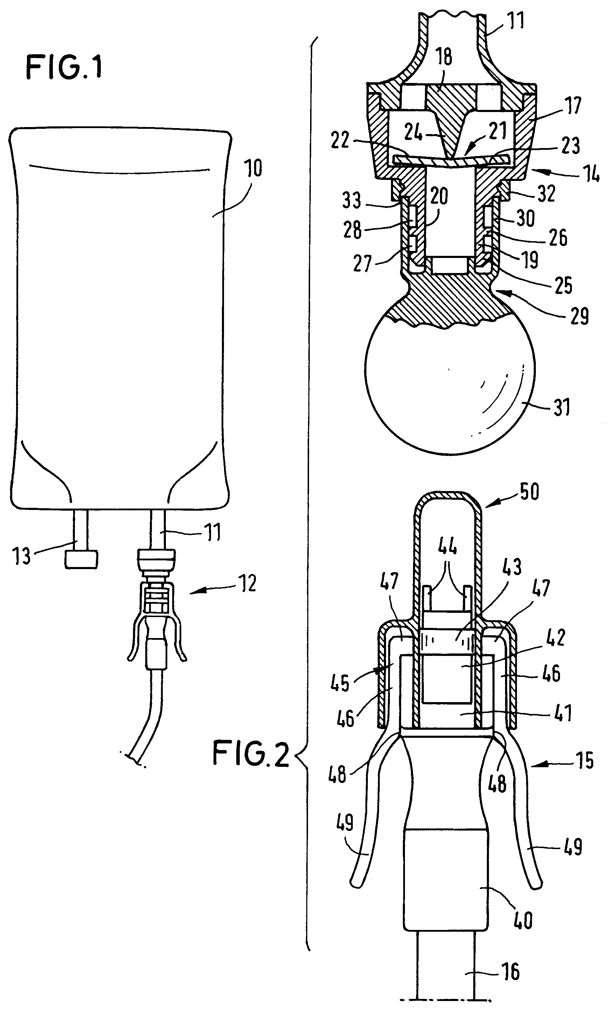

In Fig. 1 ist ein Beutel 10 dargestellt, der eine Spüllösung enthält, die in den Körper eines Patienten infundiert werden soll. Zu diesem Zweck wird der Beutel an eine Aufhängevorrichtung gehängt, wobei der Auslaßstutzen 11 nach unten weist. An dem Auslaßstutzen 11 befindet sich die Schlauchkupplung 12. Ferner ist der Beutel 10 mit einem Gummistopfen versehen, welcher das Zuspritzen eines anderen Mediums ermöglicht.

Die Schlauchkupplung 12 besteht gemäß Fig. 2 aus einem mit dem Auslaßstutzen 11 des Beutels verbundenen Kupplungselement 14 und einem Steckerteil 15, welches mit einem Überleitungsschlauch 16 verbunden ist und an das Kupplungselement 14 angekuppelt werden kann.

Das Kupplungselement 14 weist ein Gehäuse 17 auf, welches zu dem Anschlußstutzen 11 hin durch eine gelochte Wand 18 begrenzt ist, während zum entgegengesetzten Ende ein rohrförmiger Kupplungsansatz 19 absteht. Durch den Kupplungsansatz 19 erstreckt sich der zylindrische Durchflußkanal 20. An dem einen Ende des Durchflußkanals 20 befindet sich das Ventil 21 in form einer elastischen Scheibe 22, deren Rand gegen einen das Ende des Durchflußkanals 20 umgebenden Ventilsitz 23 drückt. Gegen den Mittelbereich der Scheibe 22 drückt die Spitze eines Dornes 24, so daß die Scheibe 22 leicht durchgebogen wird und mit Druck abdichtend gegen den Sitz 23 gedrückt wird.

Der Kupplungsansatz 19 weist zwei Halteteile 25,26 auf, die jeweils als umlaufender Ringkragen ausgebildet sind. Zwischen den Halteteilen 25 und 26 befindet sich eine Ringnut 27 und zwischen dem Halteteil 26 und dem Gehäuse 17 befindet sich eine weitere Ringnut 28.

Im Lieferzustand sitzt auf dem Kupplungsansatz 19 eine Verschlußkappe 29, die einen zylindrischen Kappenteil 30 und einen Handknauf 31 aufweist. Der Kappenteil 30 ist mit einem Ring 32 verbunden, welcher an dem Gehäuse 17 befestigt ist. Zwischen dem Kappenteil 30 und dem Ring 32 befindet sich eine Sollbruchstelle 33, die einen keimdichten Verschluß bildet. Auf diese Weise ist der Kupplungsansatz 19 von der Kappe 29 keimdicht umschlossen. Zum Öffnen wird die Verschlußkappe 29 gedreht, wodurch sie an der Sollbruchstelle 33 abreißt. Das Steckerteil 15 enthält einen Gehäusekörper 40, der mit dem Schlauch 16 abdichtend verbunden ist und am entgegengesetzten Ende einen Schutzkappensitz 41 aufweist. Von dem Schutzkappensitz 41 erstreckt sich ein zylindrischer Steckansatz 42 in axialer Richtung. Auf dem Steckansatz 42 befindet sich ein aufgesteckter oder aufgespritzer Dichtring 43. Von dem Ende des Steckansatzes 42 stehen Stege 44 in axialer Richtung ab. Die Stege 44 liegen einander um 180° gegenüber.

Das Steckerteil 15 enthält ferner eine Rastvorrichtung 45, die aus zwei Rastklinken 46 besteht, welche einander um 180° gegenüberliegen und mit nach innen gerichteten Rastnasen 47 ausgestattet sind. Die Rastklinken sind jeweils mit einem Kunststoffgelenk 48 mit dem Gehäuse 40 verbunden und sie setzen sich jeweils in einem Auslösehebel 49 fort, der seitlich neben dem Gehäuse 40 nach hinten ragt. Die Kunststoffgelenke 48 spannen die Rastklinken 46 so vor, daß die Rastnasen 47 gegen den Steckansatz 42 gedrückt werden. Durch manuellen Druck der Auslösehebel 49 gegen das Gehäuse 40 werden die Rastklinken 46 in die Öffnungsstellung geschwenkt.

Der Steckansatz 42 ist von einer Schutzkappe 50 umgeben, die auf dem Schutzkappensitz 41 festgeklemmt ist und den Steckansatz keimdicht umschließt. Vor Herstellung der Kupplungsverbindung wird die Schutzkappe 50 abgezogen.

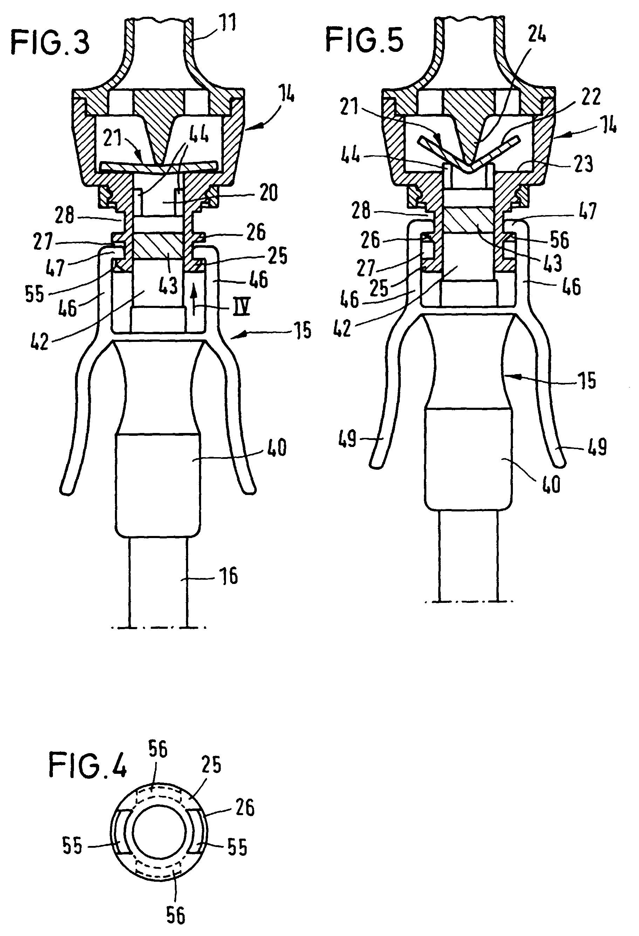

Figur 3 zeigt den ersten Kupplungszustand, den das Steckerteil 15 annimmt, wenn es zunächst vorbereitend an das Kupplungselement 14 angekuppelt wird, ohne daß jedoch das Ventil 21 geöffnet wird. Der Steckansatz 42 wird in den Durchflußkanal 20 passend eingesteckt, wobei die Dichtung 43 den Durchflußkanal nach außen hin abdichtet. Während des Einschiebens stoßen die Rastnasen 47 gegen die Einführschrägen 55 des Halteteils 25. Dadurch werden die Rastklinken 46 auseinandergedrückt bis die Rastnasen 47 in der Ringnut 27 einrasten. Ein weiteres Vorschieben wird durch das Halteteil 26 verhindert, auch dann, wenn die beiden Auslösehebel 49 betätigt werden. In diesem Zustand befinden sich die Stege 44 noch im Abstand von dem Ventil 21. Es fließt noch keine Flüssigkeit von dem Kupplungselement 14 zu dem Steckerteil 15. Durch den rohrförmigen Steckansatz 42 und das Gehäuse 40 verläuft ein (nicht dargestellter) Kanal, der mit dem Lumen des Schlauchs 16 in Verbindung steht.

In Fig. 4 sind die beiden Einführschrägen 55 erkennbar, die einander um 180° gegenüberliegend an dem ringförmigen Halteteil 25 vorgesehen sind. Das Steckerteil 15 muß daher in einer ganz bestimmten Drehposition an das Kupplungselement angesetzt werden, damit die Rastnasen 47 auf den Einführschrägen 55 gleiten.

An dem ringförmigen Halteteil 26 sind ebenfalls zwei Einführschrägen 56 vorgesehen, die jedoch gegenüber den Einführschrägen 55 um 90° versetzt angeordnet sind (Fig. 4).

Nach einer 90°-Drehung des Steckerteils 15 kann dieses von der in Fig. 3 dargestellten Position aus weiter vorgeschoben werden, so daß sein Steckansatz 42 tiefer in den Durchflußkanal 20 eindringt und die Stege 44 das Ventil 21 aufstoßen. Dadurch wird die Scheibe 22 über dem Dorn 24 V-förmig verformt, wobei sie von dem Sitz 24 abhebt und den Durchfluß von dem Gehäuse 17 in den Durchflußkanal 20 freigibt. Bei dem weiteren Einschieben des Steckansatzes 42 gleiten die Rastnasen 47 über die Einführschrägen 56, um anschließend hinter dem Halteteil 26 einzurasten. Dadurch wird das Steckerteil 15 an dem Kupplungselement 14 verriegelt. In dieser Position kann das Steckerteil 15 beliebig um seine Achse gedreht werden, ohne daß die Verriegelung sich löst. Eine solche Drehung ist auch in der in Fig. 3 dargestellten Position möglich.

Zum Lösen der Kupplung werden die beiden Auslösehebel 49 gegeneinandergedrückt. Dadurch gelangen die Rastnasen 47 außer Eingriff mit den Halteteilen 26 bzw. 25, so daß das Steckerteil 15 aus dem Kupplungselement 14 herausgezogen werden kann.

## Patentansprüche

1. Schlauchkupplung für ein medizinisches Überleitungssystem, mit
einem Kupplungselement (14), das einen Durchflußkanal (20) aufweist,
einem Steckerteil (15), das einen in den Durchflußkanal (20) abdichtend einführbaren Steckansatz (42) aufweist,
einem in dem Kupplungselement (14) angeordneten Ventil (21), das den Durchflußkanal (20) absperrt und durch Einführen des Steckansatzes (42) aufstoßbar ist,
und einem an dem Kupplungselement (14) vorgesehenen ersten Halteteil (26), welches das Einrasten einer an dem Steckerteil (15) vorgesehenen Rastvorrichtung (45) dann ermöglicht, wenn der Steckansatz (42) so weit in den Durchflußkanal (20) eingeführt ist, daß er das Ventil (21) aufstößt.

2. Schlauchkupplung nach Anspruch 1, dadurch gekennzeichnet, daß das erste Halteteil (26) ein Ringkragen ist, der mindestens eine von der Rastvorrichtung (45) überwindbare Einführschräge (56) aufweist und das Zurückziehen der eingerasteten Rastvorrichtung (45) des Steckerteils (15) verhindert.

3. Schlauchkupplung nach Anspruch 1 oder 2, dadurch gekennzeichnet, daß an dem Kupplungselement (14) ein zweites Halteteil (25) vorgesehen ist, welches das Einrasten der an dem Steckerteil (15) vorgesehenen Rastvorrichtung (45) dann ermöglicht, wenn der Steckansatz (42) abdichtend in dem Durchflußkanal (20) sitzt, das Ventil (21) jedoch noch nicht aufstößt.

4. Schlauchkupplung nach Anspruch 3, dadurch gekennzeichnet, daß das zweite Halteteil (25) ein Ringkragen ist, der mindestens eine von der Rastvorrichtung (45) überwindbare Einführschräge (55) aufweist, welche zu der Einführschräge (56) des ersten Halteteils (26) umfangsmäßig versetzt ist.

5. Schlauchkupplung nach einem der Ansprüche 1 bis 4, dadurch gekennzeichnet, daß die Rastvorrichtung (45) mindestens eine Rastklinke (46) aufweist, die in Richtung auf den Steckansatz (15) vorgespannt und mit einem Auslösehebel (49) verbunden ist.

6. Schlauchkupplung nach Anspruch 5, dadurch gekennzeichnet, daß zwei mit Auslösehebeln (49) versehene Rastklinken (46) einander gegenüberliegend vorgesehen sind.

7. Schlauchkupplung nach einem der Ansprüche 1 bis 6, dadurch gekennzeichnet, daß der Steckansatz (42) mindestens einen axial abstehenden Steg (44) zum Aufstoßen des Ventils (21) aufweist.

8. Schlauchkupplung nach einem der Ansprüche 1 bis 7, dadurch gekennzeichnet, daß das Ventil (21) aus einer elastischen Scheibe (22) besteht, die von einem Dorn (24) des Kupplungselements (14) zentral abgestützt ist und mit ihrem Rand gegen einen Sitz (23) drückt.
